(19) Europäisches Patentamt
European Patent Office
Office européen des brevets

(11) Veröffentlichungsnummer: **0 000 760**
**A1**

(12) # EUROPÄISCHE PATENTANMELDUNG

(21) Anmeldenummer: 78100554.1

(22) Anmeldetag: 31.07.78

(51) Int. Cl.²: C 07 D 317/54, A 01 N 9/24

(30) Priorität: 10.08.77 DE 2736017

(43) Veröffentlichungstag der Anmeldung: 21.02.79 Patentblatt 79/4

(84) Benannte Vertragsstaaten: BE CH DE FR GB NL

(71) Anmelder: Bayer Aktiengesellschaft
Zentralbereich Patente,Marken und Lizenzen Bayerwerk
D-5090 Leverkusen 1(DE)

(72) Erfinder: Mues, Volker, Dr
Maréestrasse 61
D-5600 Wuppertal 1(DE)

(72) Erfinder: Behrenz, Wolfgang, Dr.
Untergründemich 14
D-5063 Overath(DE)

(72) Erfinder: Rauleder, Gebhard Dr.
Cheruskerstrasse 31
D-4000 Düsseldorf(DE)

(72) Erfinder: Waldmann, Helmut, Dr.
Carl-Rumpff-Strasse 59
D-5090 Leverkusen 1(DE)

(54) **Benzodioxolderivate, Verfahren zu ihrer Herstellung und ihre Verwendung als Synergisten in insektiziden und akariziden Mitteln.**

(57) Benzodioxolderivate der Formel

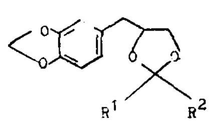

in welcher
$R^1$ und $R^2$ gleich oder verschieden sein können und für H, Alkyl, Alkenyl, Alkinyl und Aryl stehen oder
$R^1$ und $R^2$ zusammen für eine Alkylengruppe stehen, und ein Verfahren zu ihrer Herstellung.
Diese neuen Benzodioxolderivate weisen zusammen mit
A) Carbamaten und/oder
B) Carbonsäureestern, einschliesslich der natürlichen sowie synthetischen Pyrethroide, und/oder
C) Phosphorsäureestern und/oder
D) Cycloalkanen und/oder
E) Halogenalkanen
eine besonders hohe insektizide und akarizide Wirkund auf.



Croydon Printing Company Ltd.

BAYER AKTIENGESELLSCHAFT
Zentralbereich
Patente, Marken und Lizenzen

5090 Leverkusen, Bayerwerk
Rt/AB
Typ Ia

# Benzodioxolderivate, Verfahren zu ihrer Herstellung und ihre Verwendung als Synergisten in insektiziden und akariziden Mitteln

Die vorliegende Erfindung betrifft neue Benzodioxolderivate, ein Verfahren zu ihrer Herstellung sowie ihre Verwendung in insektiziden und akariziden Mitteln als Synergisten.

Es sind bereits synergistische Mischungen von Carbamaten, z.B. 2-iso-Propoxy-phenyl-N-methylcarbamat oder von Phosphorsäureestern, z.B. O,O-Diäthyl-O-[2-isopropyl-4-methyl-pyrimidin(6)yl]-thionophosphorsäureester oder von natürlichen oder synthetischen Pyrethroiden mit Piperonyläthern, wie z.B. α-[2-(2-Butoxy-äthoxy)-äthoxy]-4,5-methylendioxy-2-propyl-toluol, bekannt (vergleiche Bull. Org. Health Org. 1966, 35, Seiten 691-708; Schrader, G.: Die Entwicklung neuer insektizider Phosphorsäureester 1963, S. 158; Perkow, W.: Die Insektizide, 1966, Seiten 516-524). Doch ist die Wirksamkeit dieser synergistischen Wirkstoffkombinationen nicht befriedigend. Eine gewisse praktische Bedeutung hat bisher nur das α-[2-(2-Butoxy-äthoxy)-äthoxy]-4,5-methylendioxy-2-propyl-toluol erlangt.

Es wurden nun die neuen Benzodioxolderivate der Formel (I)

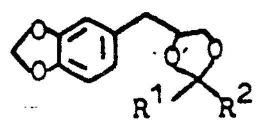

(I)

in welcher

$R^1$ und $R^2$ gleich oder verschieden sein können und für H, Alkyl, Alkenyl, Alkinyl und Aryl stehen oder

$R^1$ und $R^2$ zusammen für eine Alkylengruppe stehen

gefunden.

Es wurde außerdem gefunden, daß diese Verbindungen hergestellt werden können, indem man eine Verbindung der Formel II

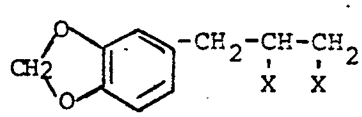

(II)

in welcher

X für OH oder beide Reste X für -O- stehen,

mit einer Carbonylverbindung der allgemeinen Formel III

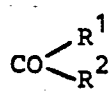

(III)

in welcher

$R^1$ und $R^2$ die oben angegebene Bedeutung haben,

gegebenenfalls in Gegenwart eines Verdünnungsmittels umsetzt.

Es wurde außerdem gefunden, daß diese neuen Benzodioxolderivate zusammen mit

A) Carbamaten und/oder

B) Carbonsäureestern, einschließlich der natürlichen sowie synthetischen Pyrethroide, und/oder

C) Phosphorsäureestern und/oder

D) Cycloalkanen und/oder

E) Halogenalkanen

eine besonders hohe insektizide und akarizide Wirkung aufweisen.

Die synergistische Wirkung der Verbindungen der allgemeinen Formel (I) zeigt sich bevorzugt bei den

A) Carbamaten der allgemeinen Formel (IV)

$$\begin{matrix}R^4\\R^5\end{matrix}\!\!>N-CO-OR^3 \qquad (IV)$$

in welcher

$R^3$ für Aryl, heterocyclische Reste oder Oximrest steht,

$R^4$ für Wasserstoff oder Alkyl mit 1 bis 4 Kohlenstoffatomen steht und

$R^5$ für Alkyl, Alkylcarbonyl mit 1 bis 5 Kohlenstoffatomen im Alkylrest, der gegebenenfalls auch durch Hydroxy oder Methylthio substituiert sein kann, oder den Rest -S-Z steht, wobei

Z für einen gegebenenfalls durch Halogen substituierten aliphatischen Rest mit 1 bis 4 Kohlenstoffatomen, insbesondere $CCl_3$ und $CF_3$ sowie für einen gegebenenfalls bevorzugt durch Nitril, Halogen, insbesondere Chlor, Methyl, Trihalogenmethyl, Trifluormethylmercapto oder Nitro substituierten Arylrest, insbesondere Phenyl, oder für Methoxycarbonyl oder für den Rest $W-SO_2-\underset{R^4}{\underset{|}{N}}-$ steht, wobei

W für Alkyl, Halogen-alkyl oder Alkylamino, Dialkylamino oder einen gegebenenfalls bevorzugt durch Halogen, Trihalogenmethyl, Nitril, Methyl oder Nitro substituierten Arylrest steht.

Besonders bevorzugt sind Carbamate in denen

$R^3$ für Phenyl oder Naphtyl steht, die gegebenenfalls substituiert sind durch Alkyl, Alkenyl, Alkoxy, Alkylmercapto oder Alkylthioalkylen mit jeweils 1 bis 5 Kohlenstoffatomen, Dialkylamino, Dialkenylamino mit bis zu 3 Kohlenstoffatomen je Alkyl-bzw. Alkenylteil, Halogen, insbesondere Chlor, Dioxolanyl oder den Rest $-N{=}CH-N(C_{1-4}\text{-Alkyl})_2$ steht.

Weiterhin sind besonders bevorzugt Carbamate, in denen

$R^3$ für 2,3-Dihydrobenzofuranyl, Benzodioxol, Benzothienyl, Pyrimidinyl oder Pyrazolyl steht, die gegebenenfalls durch

BAD ORIGINAL

$C_{1-4}$-Alkyl, insbesondere Methyl, oder Dialkylamino mit 1 bis 4 Kohlenstoffatomen je Alkylteil substituiert sind.

Weiterhin sind besonders bevorzugt Carbamate, in denen $R^3$ für einen Oximrest der allgemeinen Formel (IVa)

$$-N{=}C\begin{matrix}R^6\\ R^7\end{matrix} \qquad (IVa)$$

steht, in welcher

$R^6$ und $R^7$ gleich oder verschieden sind und für Alkyl, Cycloalkyl, Alkenyl, Alkinyl, Alkoxy, Alkylmercapto, Alkoxycarbonyl, Carbonylamid, Alkylmercaptoalkyl, mit jeweils bis zu 5 Kohlenstoffatomen, Nitril, Aryl, insbesondere Phenyl, einen gegebenenfalls substituierten heterocyclischen Rest oder für Alkyl, das durch einen heterocyclischen Rest substituiert ist oder gemeinsam einen gegebenenfalls durch $C_{1-4}$-Alkyl substituierten Dioxolanyl oder Dithiolanylrest stehen;

Besonders erwähnt seien folgende Carbamate
2-Methylphenyl-, 2-Äthylphenyl-, 2-n-Propylphenyl-, 2-Methoxyphenyl-, 2-Äthoxyphenyl-, 2-iso-Propoxyphenyl-, 4-Methylphenyl-, 4-Äthylphenyl-, 4-n-Propylphenyl-, 4-Methoxyphenyl-, 4-Äthoxyphenyl-, 4-n-Propoxyphenyl-, 3,4,5-Trimethylphenyl-, 1-Naphthyl-, 2.3-Dihydro-2,2-dimethyl-7-benzofuranyl-, 2-[1,3-Dioxolan(2)yl-phenyl]- bzw. 2,2-Dimethyl-1,3-benzodioxol-(4)yl-N-methyl-carbamat und die entsprechenden -N-methyl-N-acetyl-, -N-methyl-N- trifluormethylthio-, -N-methyl-N-dichlormonofluormethylthio- bzw. -N-methyl-N-dimethylaminothiocarbamate.

B) Carbonsäureestern der allgemeinen Formel (V)

$$R^8-CO-O-\overset{\overset{R^9}{|}}{C}H-R^{10} \qquad (V)$$

in welcher

$R^8$ für Alkyl, Aralkyl, Aryl oder Cycloalkyl steht, die gegebenenfalls substituiert sein können und

$R^9$ für Wasserstoff, Alkyl, Halogenalkyl, Alkenyl, Alkinyl oder Nitril steht und

$R^{10}$ für Aryl oder einen Heterocyclus steht, oder gemeinsam mit $R^9$ einen gegebenenfalls substituierten Cyclopentenonring bildet.

Besonders bevorzugt sind Carbonsäureester, in denen $R^8$ für Alkyl mit 1 bis 6 Kohlenstoffatomen, das gegebenenfalls durch gegebenenfalls substituiertes Phenyl substituiert ist, Cyclopropyl, das gegebenenfalls durch Alkyl,Alkenyl, Halogenalkyl oder Halogenalkenyl mit jeweils bis zu 6 Kohlenstoffatomen substituiert ist, oder für Phenyl, das gegebenenfalls substituiert ist, steht. Bevorzugt sind Carbonsäureester, in denen $R^9$ für Wasserstoff, Alkyl mit 1 bis 6 Kohlenstoffatomen, Halogenalkyl mit 1 bis 4 Kohlenstoffatomen und bis zu 3 Halogenatomen, Nitril oder Äthinyl steht.

Weiter sind besonders bevorzugt Carbonsäureester, in denen $R^{10}$ für Phenyl steht, das gegebenenfalls durch $C_{1-4}$-Alkyl, Halogen, insbesondere Fluor oder Chlor, gegebenenfalls substituiertes Phenoxy; sowie gegebenenfalls substituiertes Benzyl substituiert ist, ferner für Furanyl, Tetrahydrophthalimido, Benzodioxol, die gegebenenfalls durch Halogen, insbesondere Chlor, Alkyl oder Alkenyl mit bis zu

4-Kohlenstoffatomen oder Benzyl substituiert sind, steht, und ferner für Cyclopentenon steht, das gegebenenfalls durch $C_{1-4}$-Alkyl, Furfuryl, $C_{1-5}$-Alkenyl substituiert ist.

Weiter sind besonders bevorzugt die natürlich vorkommenden Pyrethroide;

Besonders erwähnt seien folgende Carbonsäureester

Essigsäure-[1-(3,4-dichlorphenyl)-2,2,2-trichloräthyl]-ester, 2,3,4,5-Tetrahydrophthalimidomethylchrysanthemat und (5-Benzyl-3-furyl)-methyl-2,2-dimethyl-3-(2-methyl-propenyl)-cyclopropan-carboxylat.

C) Phosphorsäureestern der allgemeinen Formel (VI)

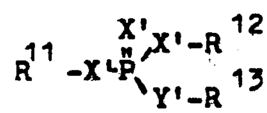
(VI)

in welcher

X' unabhängig voneinander für O oder S steht und

Y' für O, S, -NH- oder für eine direkte Bindung zwischen dem zentralen P-Atom und dem Rest $R^{13}$ steht und

$R^{11}$ und $R^{12}$ gleich oder verschieden sind und für Alkyl oder Aryl stehen und

$R^{13}$ für Alkyl, Aryl, Heteroaryl, Aralkyl, Alkenyl, Dioxanyl, oder einen Oximrest oder für den gleichen Rest steht, an den es gebunden ist.

Besonders bevorzugt sind Phosphorsäureester, in denen

$R^{11}$ und $R^{12}$ gleich oder verschieden sind und für $C_{1-4}$-Alkyl oder Phenyl stehen,

$R^{13}$ für Alkyl mit 1 bis 4 Kohlenstoffatomen steht, das gegebenenfalls durch Halogen, Hydroxyl, Nitril, gegebenfalls substituiertes Phenyl, Carbonylamid, Sulfonylalkyl, Sulfoxyalkyl, Carbonylalkyl, Alkoxy, Alkylmercapto, Alkoxycarbonyl, substituiert ist, für Alkenyl mit bis zu 4 Kohlenstoffatomen, das gegebenenfalls durch Halogen, gegebenenfalls halogensubstituiertes Phenyl oder Alkoxycarbonyl substituiert ist, oder für den Oximrest der allgemeinen Formel (IVa)

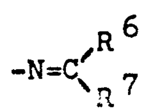

(IVa)

wobei $R^6$ und $R^7$ die oben angegebene Bedeutung besitzen, insbesondere jedoch für Cyan oder Phenyl stehen,

$R^{13}$ steht ferner für Dioxanyl, das durch denselben Rest substituiert ist, an den $R^{13}$ gebunden ist, oder $R^{13}$ steht für den gleichen Rest, an den er gebunden ist, oder $R^{13}$ steht für Phenyl, das gegebenenfalls durch Methyl, Nitro, Nitril, Halogen, Methylthio substituiert ist; $R^{13}$ steht außerdem besonders bevorzugt für gegebenenfalls durch $C_{1-4}$-Alkyl, Halogen substituierte Heteroaromaten, wie Pyridin, Chinolin, Chinoxalin, Pyrimidin, Diazinon, Benzo-1,2,4-triazin.

Besonders erwähnt seien folgende Phosphorsäureester
0,0-Dimethyl- bzw. 0,0-Diäthyl-0-(2,2-dichlor- bzw. 2,2-dibromvinyl)-phosphorsäureester,
0,0-Diäthyl-0-(4-nitro-phenyl)-thionophosphorsäureester,
0,0-Dimethyl-0-(3-methyl-4-methylthio)-thionophosphorsäureester,
0,0-Dimethyl-0-(3-methyl-4-nitro)-thionophosphorsäureester,
0-Äthyl-S-n-propyl-0-(2,4-dichlorphenyl)-thionophosphorsäureester,
0-Äthyl-S-n-propyl-0-(4-methylthio-phenyl)-thionophosphorsäureester,
0,0-Dimethyl-S-[4-oxo-1,2,3-benzotriazin(3)yl-methyl]-thionothiolphosphorsäureester,
0-Methyl-0-[2-iso-propyl-6-methoxy-pyrimidin(4)yl]-thionomethanphosphonsäureester,
0,0-Diäthyl-0-[2-iso-propyl-6-methyl-pyrimidin(4)yl]-thionophosphorsäureester,
0,0-Diäthyl-0-[3-chlor-4-methyl-cumarin(7)yl]-thionophosphorsäureester,
0,0-Dimethyl-2,2,2-trichlor-1-hydroxy-äthan-phosphonsäureester,
0,0-Dimethyl-S-(methylcarbamoylmethyl)-thionophosphorsäureester.

D) Cycloalkanen der Formel (VII)

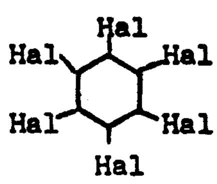

(VII)

in welcher
Hal Halogen, vorzugsweise Chlor, bedeutet.

Besonders erwähnt sei 1,2,3,4,5,6-Hexachlorohexan.

E) Halogenalkanen der Formel (VIII)

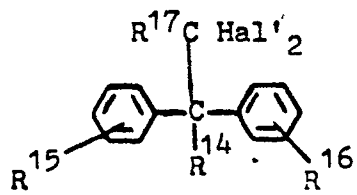

(VIII)

in welcher

Hal' für Chlor oder Brom

$R^{14}$ für Wasserstoff oder Hydroxyl,

$R^{15}$ und $R^{16}$ gleich oder verschieden sind und für Halogen, Alkyl oder Alkoxy und

$R^{17}$ für Wasserstoff oder Halogen stehen.

Besonders bevorzugt sind Halogenalkane, in denen

$R^{14}$ Wasserstoff oder Hydroxyl bedeutet,

$R^{15}$ und $R^{16}$ für gleiches Halogen, Alkyl bzw. Alkoxy mit 1 bis 4 Kohlenstoffatomen je Alkyl- bzw. Alkoxyrest stehen und

$R^{17}$ Halogen bedeutet.

Besonders zu erwähnen seien folgende Halogenalkane

1,1,1-Trichlor-2,2-bis-(4-chlor- bzw. 4-methoxyphenyl)-äthan, 1,1,1-Trichlor-2-hydroxy-2,2-bis-(4-chlorphenyl)-äthan und 1,1-Dichlor-2,2-bis-(4-äthylphenyl)-äthan.

Überraschenderweise ist die insektizide und/oder akarizide Wirkung solcher Wirkstoffkombinationen wesentlich höher als die Wirkung der Einzelkomponenten bzw. die Summe der Wirkungen der Einzelkomponenten. Sie ist ferner wesentlich höher als die Wirkung von Wirkstoffkombination mit dem bekannten Synergisten Piperonylbutoxyd.

Außerdem zeigen die erfindungsgemäß verwendbaren Benzodioxolderivate ausgezeichnete synergistische Wirksamkeit nicht nur bei einer Wirkstoffklasse, sondern bei Wirkstoffen aus den verschiedensten chemischen Stoffgruppen.

Somit stellen die erfindungsgemäßen Benzodioxolderivate eine wertvolle Bereicherung der Technik dar.

Die neuen Benzodioxolderivate sind durch die Formel (I) allgemein definiert. Vorzugsweise stehen in der Formel (I) jedoch

$R^1$ und $R^2$ für Wasserstoff, geradkettiges oder verzweigtes Alkyl bzw. Alkenyl mit bis zu 10 C-Atomen, sowie für gegebenenfalls substituiertes Phenyl

oder

$R^1$ und $R^2$ stehen zusammen für eine geradkettige oder verzweigte Alkylkette mit bis zu 10 C-Atomen.

Als Beispiele seien im einzelnen genannt:
5-(3.4-Methylendioxybenzyl)-dioxolan
2-Methyl-5-(3.4-methylendioxybenzyl)-dioxolan

2-Äthyl-5-(3.4-methylendioxybenzyl)-dioxolan
2-n-Propyl-5-(3.4-methylendioxybenzyl)-dioxolan
2-i-Propyl-5-(3.4.-methylendioxybenzyl)-dioxolan
2-n-Butyl-5-(3.4.-methylendioxybenzyl)-dioxolan
2-i-Butyl-5-(3.4-methylendioxybenzyl)-dioxolan
2-t-Butyl-5-(3.4-methylendioxybenzyl)-dioxolan
2-Allyl-5-(3.4-methylendioxybenzyl)-dioxolan
2-Methallyl-5-(3.4-methylendioxybenzyl)-dioxolan
2-Crotyl-5-(3.4-methylendioxybenzyl)-dioxolan
2-Äthinyl-5-(3.4-methylendioxybenzyl)-dioxolan
2-Phenyl-5-(3.4-methylendioxybenzyl)-dioxolan
2-(3.4-Methylendioxyphenyl)-5-(3.4-methylendioxybenzyl)-dioxolan
2,2-Dimethyl-5-(3.4-methylendioxybenzyl)-dioxolan
2-Methyl-2-äthyl-5-(3.4-methylendioxybenzyl)-dioxolan
2-Methyl-2-n-propyl-5-(3.4-methylendioxybenzyl)-dioxolan
2-Methyl-2-i-propyl-5-(3.4-methylendioxybenzyl)-dioxolan
2-Methyl-2-n-butyl-5-(3.4-methylendioxybenzyl)-dioxolan
2-Methyl-2-i-butyl-5-(3.4-methylendioxybenzyl)-dioxolan
2-Methyl-2-t-butyl-5-(3.4-methylendioxybenzyl)-dioxolan
2,2-Diäthyl-5-(3.4-methylendioxybenzyl)-dioxolan
2,2-Di-n-propyl-5-(3.4-methylendioxybenzyl)-dioxolan
2,2-Di-n-butyl-5-(3.4-methylendioxybenzyl)-dioxolan
2-Methyl-2-phenyl-5-(3.4-methylendioxybenzyl)-dioxolan
2-Spirocyclopentan-5-(3.4-methylendioxybenzyl)-dioxolan
2-Spirocyclohexan-5-(3.4-methylendioxybenzyl)-dioxolan
2-(2.4.4-Trimethyl-spirocyclohexan)-5-(3,4-methylendioxybenzyl)-dioxolan.

Bei den als Ausgangsverbindungen zur Herstellung der erfindungsgemäßen Benzodioxolderivate dienenden Verbindungen der Formel II handelt es sich um 3-(3,4-Methylendioxyphenyl)-propandiol-1,2 und um 3,4-Methylendioxybenzyläthylenoxid. Beide Verbindungen sind bekannt, ebenso wie die Carbonylverbindungen der Formel III.

Das Verfahren zur Herstellung der erfindungsgemäßen Verbindungen wird gegebenenfalls unter Mitverwendung geeigneter Lösungs- oder Verdünnungsmittel durchgeführt. Als solche kommen praktisch alle inerten organischen Solventien infrage. Hierzu gehören insbesondere aliphatische und aromatische, gegebenenfalls chlorierte, Kohlenwasserstoffe, wie Benzol, Toluol, Xylol, Methylenchlorid, Chloroform und Tetrachlorkohlenstoff.

Für den Fall, daß die X für OH stehen, arbeitet man in Gegenwart von sauren Katalysatoren, wie z.B. Schwefelsäure oder p-Toluolsulfonsäure und für den Fall, daß die beiden X für ein Sauerstoffatom stehen, arbeitet man in Gegenwart eines Katalysators, wie Bortrifluorid.

Die Reaktionstemperatur kann innerhalb eines größeren Bereichs variiert werden. Im allgemeinen arbeitet man zwischen 25 und 15o°C, vorzugsweise zwischen 7o und 12o°C.

Die Umsetzung läßt man im allgemeinen bei Normaldruck ablaufen.

Zur Durchführung des Verfahrens setzt man bei Verwendung von 3-(3,4-Methylendioxyphenyl)-propandiol-1,2 als Ausgangsprodukt die Verbindungen vorzugsweise in äquimolaren Mengen ein. Ein Überschuß der einen oder anderen Reaktionskomponente bringt keine wesentlichen Vorteile. Im allgemeinen werden die beiden Reaktionskomponenten in einem der angegebenen Lösungsmittel in Gegenwart eines sauren Katalysators bei den angegebenen Temperaturen umgesetzt. Das dabei entstehende Wasser wird durch azeotrope Destillation entfernt.

Verwendet man 3,4-Methylendioxybenzyläthylenoxyd als Ausgangskomponente, so ist eine bevorzugte Ausführungsform die, daß

die Carbonylkomponente im Überschuß eingesetzt wird und so als Reaktionskomponente und Lösungsmittel dient. Die Umsetzung erfolgt vorzugsweise in Gegenwart eines der angegebenen Katalysators bei den angegebenen Temperaturen. Die Aufarbeitung nach beendeter Reaktion erfolgt wie üblich durch Ausschütteln mit einem organischen Lösungsmittel, Waschen, Trocknen der organischen Phase und Abdestillieren des Lösungsmittels.

Die erfindungsgemäßen Verbindungen werden durch ihren Siedepunkt charakterisiert.

Wie bereits erwähnt, zeigen Wirkstoffkombinationen der erfindungsgemäßen Benzodioxolderivate der Formel (I) mit Carbamaten, Carbonsäureestern, Phosphorsäureestern, Cycloalkanen und Halogenalkanen eine hervorragende Wirkungssteigerung gegenüber den Einzelwirkstoffen bzw. gegenüber deren Summe.

Die Gewichtsverhältnisse der Wirkstoffgruppen können dabei in relativ großen Bereichen schwanken. Im allgemeinen werden die Benzodioxolderivate mit den übrigen Wirkstoffen im Verhältnis 0,1:10 bis 10:0,1 eingesetzt. Als besonders geeignet haben sich jedoch Mischverhältnisse von 0,5:1,0 bis 3,0:1,0 erwiesen. Diese Wirkstoffkombinationen bewirken nicht nur eine schnelle knock-down-Wirkung, sondern bewirken auch die nachhaltige Abtötung von tierischen Schädlingen, insbesondere Insekten und Spinnentieren, die in der Landwirtschaft, in Forsten, im Vorrats- und Materialschutz sowie auf dem Hygienesektor vorkommen. Sie sind gegen normale sensible und resistente Arten sowie gegen alle oder einzelne Entwicklungsstadien wirksam. Zu den oben erwähnten Schädlingen gehören:

Aus der Ordnung der Isopoda zum Beispiel Oniscus asellus, Armadillidium vulgare, Porcellio scaber.

Aus der Ordnung der Diplopoda zum Beispiel Blaniulus guttulatus.

Aus der Ordnung der Chilopoda zum Beispiel Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp.,

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis billobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis,

Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.,

Aus der Ordnung der Diptera z.B. Aëdes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp. .
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp. .

Die Wirkstoffkombinationen können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspension-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Wärmenebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline,

chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Tägerstoffe: natürliche Gesteinsmehle, wie Kaoline, Tonerden, Talkum, Kreide, Quarz, Attapulgit, Montmorrillonit oder Diatomeenerde und synthetische Gesteinsmehle, wie hochdisperse Kieselsäure, Aluminiumoxid und Silikate; als feste Trägerstoffe für Granulate: gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehle, Kokosnußschalen, Maiskolben und Tabakstengel; als Emulgier- und/oder schaumerzeugende Mittel: nichtionogene und anionische Emulgatoren, wie Polyoxyäthylen-Fettsäure-Ester, Polyoxyäthylen-Fettalkohol-Äther, z.B. Alkylaryl-polyglykol-äther, Alkylsulfonate, Alkylsulfate, Arylsulfonate sowie Eiweißhydrolysate; als Dispergiermittel: z.B. Lignin-, Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die Anwendung der erfindungsgemäßen Wirkstoffkombinationen erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen vairieren. Die Wirkstoffkonzentration der Anwendungsformen kann von 0,0000001 bis zu 100 Gew.-% Wirkstoff, vorzugsweise zwischen 0,01 und 10 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffkombinationen durch eine hervorragende Residualwirkung auf Holz und Ton sowie durch eine gute Alkalistabilität auf gekälkten Unterlagen aus.

Beispiel A

$LT_{100}$ - Test

Testtiere: Musca domestica, Stamm Weymanns
(gegen Carbamate und Phosphorsäureester resistent)

Lösungsmittel: Aceton

Von den Wirkstoffen, Synergisten und Gemischen aus Wirkstoffen und Synergisten werden Lösungen hergestellt und 2,5 ml davon in Petrischalen auf Filterpapierscheiben von 9,5 cm Durchmesser pipetiert. Das Filterpapier saugt die Lösungen auf. Die Petrischalen bleiben so lange offen stehen, bis das Lösungsmittel vollständig verdunstet ist. Anschließend gibt man 25 Testtiere in die Petrischalen und bedeckt sie mit einem Glasdeckel.

Der Zustand der Testtiere wird bis zu 6 Stunden laufend kontrolliert. Es wird diejenige Zeit ermittelt, die für eine 100 %ige knock-down-Wirkung erforderlich ist. Wird die $LT_{100}$ nach 6 Stunden nicht erreicht, wird der %-Satz der knock down gegangenen Testtiere festgestellt.

Konzentrationen der Wirkstoffe, Synergisten und Gemische und ihre Wirkungen gehen aus der nachfolgenden Tabelle hervor.

T a b e l l e

LT 100-Test mit phosphorsäureester-resistenten Musca domestica (Stamm Weymanns)

| Wirkstoffe bzw. ( ) Kennbuchstabe | Synergisten ( ) Beispiel-Nr. | Konzentrationen in % | LT 100 nach Minuten |
|---|---|---|---|
| 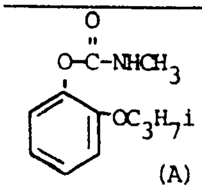 (A) | | 1,0 | 360' = 0 % |
| 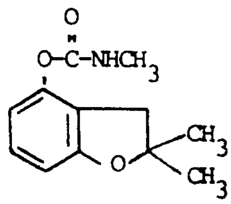 (B) | | 1,0 | 360' = 0 % |
| 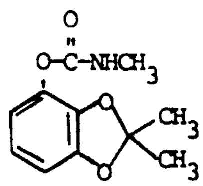 (C) | | 1,0 | 360' = 5 % |
| 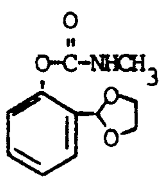 (D) | | 1,0 | 360' = 0 % |
| 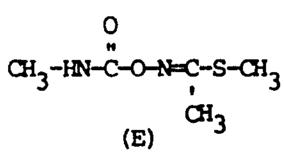 (E) | | 0,04 | 360' = 35 % |

<u>Tabelle</u> (Forsetzung)

| Wirkstoff bzw. ( ) Kennbuchstabe | Synergisten ( ) Beispiel Nr. | Konzentration in % | LT 100 nach Minuten |
|---|---|---|---|
| Pyrethrine als 25 %iger Extrakt (F) | | 0,04 | 360' = 80 % |
| 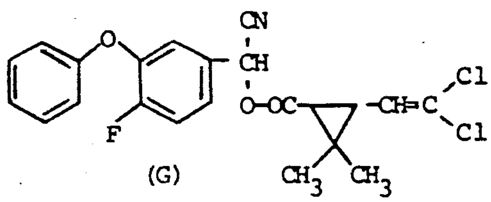 (G) | | 0,0016 | 6 h = 50 % |
| 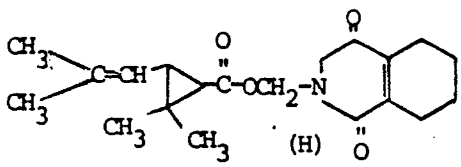 (H) | | 0,2 | 180' |
| 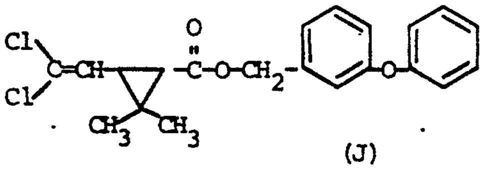 (J) | | 0,04 | 75' |
| 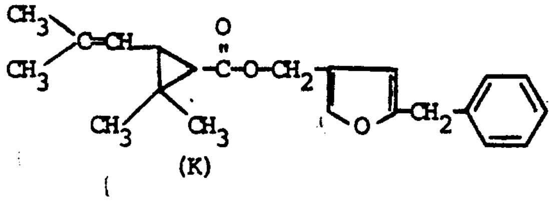 (K) | | 0,04 | 150' |
| 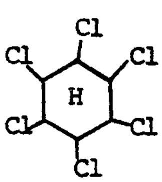 (L) | | 1,0 | 360' = 90 % |

Tabelle (Fortsetzung

| Wirkstoff ( ) Kennbuchstabe bzw. Synergisten ( ) Beispiel Nr. | Konzentration in % | LT 100 nach Minuten |
|---|---|---|
| 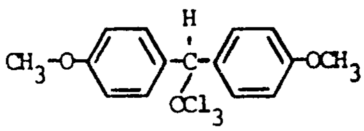 (M) | 1,0 | 360' = 5 % |
| 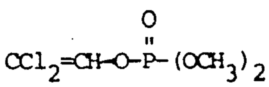 (N) | 0,008 | 240' |
| 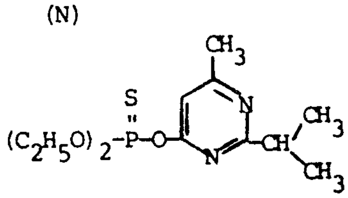 (O) | 0,04 | 360' = 5 % |
| 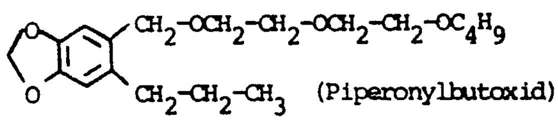 (Piperonylbutoxid) | 1,0 | 360' = 0 % |
| 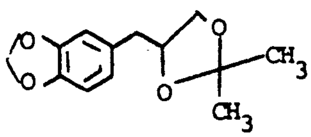 (1) | 0,2 | 360' = 70 % |
| 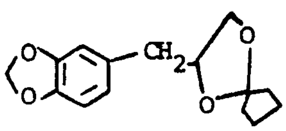 (2) | 0,2 | 360' = 0 % |
| 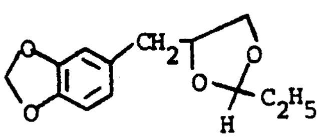 (3) | 0,2 | 360' = 0 % |

<u>Tabelle</u> (Fortsetzung)

| Wirkstoff bzw. ( ) Kennbuchstabe | Synergisten ( ) Beispiel Nr. | Konzentration in % | LT 100 nach Minuten |
|---|---|---|---|
| 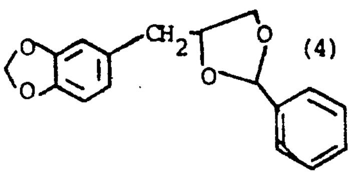 | (4) | 1,0 | 360' = 0 % |
| 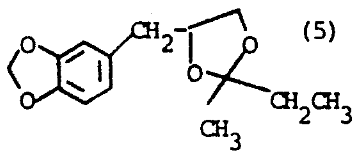 | (5) | 0,2 | 310' = 5 % |
| 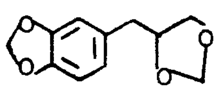 | (6) | 1,0 | 360' = 0 % |
| 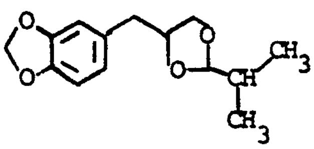 | (7) | 1,0 | 360' = 0 % |
| 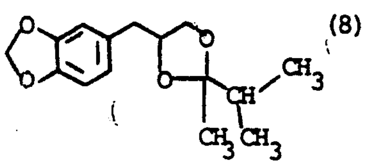 | (8) | 1,0 | 360' = 0 % |
| 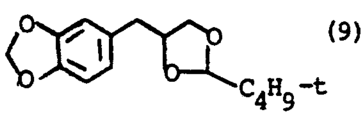 | (9) | 1,0 | 360' = 0 % |

| Wirkstoffe (Kennbuchstabe) | + Synergisten (Beispiel-Nr.) | Konzentrationen in % | LT 100 nach Minuten |
|---|---|---|---|
| A | + Piperonylbutoxid | 0,2 + 0,2 | 360' = 90 % |
| A | + 1 | 0,04 + 0,04 | 150' |
| A | + 3 | 0,2 + 0,2 | 180' |
| A | + 6 | 0,04 + 0,04 | 150' |
| A | + 7 | 0,2 + 0,2 | 240' |
| A | + 8 | 0,2 + 0,2 | 240' |
| A | + 9 | 0,2 + 0,2 | 180' |
| B | + Piperonylbutoxid | 1,0 + 1,0 | 360' = 90 % |
| B | + 1 | 0,04 + 0,04 | 120' |
| B | + 2 | 0,2 + 0,2 | 360' |
| B | + 3 | 0,04 + 0,04 | 240' |
| B | + 5 | 0,04 + 0,04 | 210' |
| C | + Piperonylbutoxid | 1,0 + 1,0 | 360' = 75 % |
| C | + 1 | 0,04 + 0,04 | 150' |
| C | + 3 | 1,0 + 1,0 | 180' |
| C | + 5 | 0,2 + 0,2 | 210' |
| D | + Piperonylbutoxid | 1,0 + 1,0 | 360' = 70 % |
| D | + 1 | 0,2 + 0,2 | 240' |
| D | + 3 | 1,0 + 1,0 | 180' |
| E | + Piperonylbutoxid | 0,04 + 0,04 | 360' = 95 % |
| E | + 1 | 0,04 + 0,04 | 150' |
| E | + 2 | 0,04 + 0,04 | 240' |
| E | + 3 | 0,04 + 0,04 | 150' |
| E | + 5 | 0,04 + 0,04 | 240' |
| F | + 1 | 0,04 + 0,04 | 45 ' |
| F | + 2 | 0,04 + 0,04 | 60 ' |
| F | + 3 | 0,04 + 0,04 | 60 ' |
| F | + 4 | 0,04 + 0,04 | 60 ' |
| F | + 5 | 0,04 + 0,04 | 60 ' |
| G | + Piperonylbutoxid | 0,0016 + 0,0016 | 360' = 70 % |
| G | + 3 | 0,0016 + 0,0016 | 105' |
| G | + 4 | 0,0016 + 0,0016 | 240' |
| H | + Piperonylbutoxid | 0,04 + 0,04 | 90 ' |
| H | + 1 | 0,04 + 0,04 | 60 ' |
| H | + 2 | 0,04 + 0,04 | 45 ' |
| H | + 5 | 0,04 + 0,04 | 45 ' |

| Wirkstoffe (Kennbuchstabe) | + | Synergisten (Beispiel-Nr.) | Konzentrationen in % | LT 100 nach Minuten |
|---|---|---|---|---|
| J | + | Piperonylbutoxyid | 0,04 + 0,04 | 75 ' |
| J | + | 2 | 0,04 + 0,04 | 60 ' |
| J | + | 5 | 0,04 + 0,04 | 45 ' |
| K | + | 1 | 0,04 + 0,04 | 30 ' |
| K | + | 2 | 0,04 + 0,04 | 30 ' |
| K | + | 3 | 0,04 + 0,04 | 45 ' |
| K | + | 4 | 0,04 + 0,04 | 45 ' |
| K | + | 5 | 0,04 + 0,04 | 60 ' |
| L | + | Piperonylbutoxid | 1,0 + 1,0 | 360' |
| L | + | 3 | 1,0 + 1,0 | 150' |
| L | + | 5 | 1,0 + 1,0 | 90' |
| M | + | Piperonylbutoxid | 1,0 + 1,0 | 360' = 45 % |
| M | + | 1 | 1,0 + 1,0 | 210' |
| N | + | Piperonylbutoxid | 0,008 + 0,008 | 240' |
| N | + | 1 | 0,008 + 0,008 | 105' |
| N | + | 5 | 0,008 + 0,008 | 105' |
| O | + | Piperonylbutoxid | 0,04 + 0,04 | 360' = 95 % |
| O | + | 5 | 0,04 + 0,04 | 120' |

Allgemeine Herstellungsverfahren:

a) 0,1 Mol 3-(3.4-Methylendioxyphenyl)-propandiol-1.2 wird mit 0,1 Mol der entsprechenden Carbonylverbindung mit 0,5 g p-Toluolsulfonsäure in 300 ml Benzol am Wasserabscheider gekocht bis zur Beendigung der Reaktion. Man kühlt ab, wäscht zur Entfernung des Katalysators mit verdünnter Natronlauge, dann mit Wasser neutral, trocknet über $Na_2SO_4$, zieht das Lösungsmittel ab und destilliert.

b) 0,1 Mol 3,4-Methylendioxybenzyl-äthylenoxid wird mit 0,5 Mol Carbonylverbindung vermischt. Bei der Zugabe weniger Tropfen Bortrifluorid-ätherat tritt eine starke exotherme Reaktion ein, die durch Kühlung mit einem Eisbad zwischen 50 und 100°C gehalten werden kann. Nach Abklingen rührt man noch 1 Stunde bei 60°C nach, nimmt mit Toluol auf, wäscht mit Wasser neutral, trocknet über $Na_2SO_4$, zieht das Lösungsmittel ab und destilliert.

Nach diesen Herstellungsverfahren werden folgende Verbindungen erhalten:

| Beispiel-Nr. | $R^1$ | $R^2$ | Herstellungsweg | Siedepunkt [°C/mm] | Ausbeute [%] |
|---|---|---|---|---|---|
| 1 | $CH_3$ | $CH_3$ | b | 140/3 | 72 |
| 2 | $-CH_2-CH_2$— | $CH_2-CH_2$ | b | 174/3 | 71 |
| 3 | H | $C_2H_5$ | b | 162/4 | 71 |
| 4 | H | $C_6H_5$ | b | 230/3 | 27 |
| 5 | $CH_3$ | $C_2H_5$ | b | 160/4 | 48 |
| 6 | H | | a | 132/3 | 56 |
| 7 | H | $i-C_3H_7$ | b | 120/0,01 | 49 |
| 8 | $CH_3$ | $i-C_3H_7$ | b | 124/0,01 | 27 |
| 8 | $CH_3$ | $i-C_3H_7$ | a | 124/0,01 | 59 |
| 9 | H | $t-C_4H_9$ | b | 122/0,01 | 40 |
| 10 | H | $n-C_3H_7$ | b | 124/0,01 | 57 |
| 11 | H | $3,4-OCH_2O-C_6H_3$ | b | 250/3 | 28 |
| 12 | $CH_3$ | $n-C_3H_7$ | b | 162/3 | 33 |
| 13 | $CH_3$ | $t-C_4H_9$ | b | 100/0,1 | 35 |
| 14 | $CH_3$ | $C_6H_5$ | b | 206/4 | 32 |
| 15 | $C_2H_5$ | $C_2H_5$ | b | 160/3 | 21 |
| 15 | $C_2H_5$ | $C_2H_5$ | a | 160/3 | 63 |
| 16 | $-CH_2-CH_2-CH_2-CH_2-CH_2-$ | | b | 155/0,01 | 63 |
| 17 | 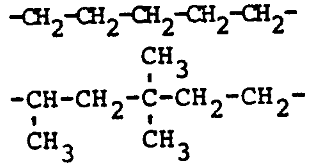 | | b | 186/3 | 52 |

## Patentansprüche

1. Benzodioxolderivate der Formel (I)

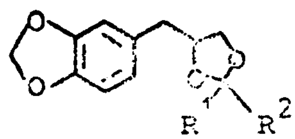

in welcher

$R^1$ und $R^2$ gleich oder verschieden sein können und für H, Alkyl, Alkenyl, Alkinyl und Aryl stehen oder

$R^1$ und $R^2$ zusammen für eine Alkylengruppe stehen.

2. Verfahren zur Herstellung der Benzodioxolderivate der Formel I gemäß Anspruch 1, dadurch gekennzeichnet, daß man eine Verbindung der Formel II

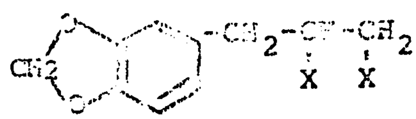

(II)

in welcher

X für OH oder beide Reste X für -O- stehen

mit einer Carbonylverbindung der allgemeinen Formel III

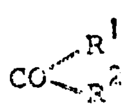

(III)

in welcher

$R^1$ und $R^2$ die oben angegebene Bedeutung haben, gegebenenfalls in Gegenwart eines Verdünnungsmittels umsetzt.

3. Insektizide und akarizide Mittel, gekennzeichnet durch einen Gehalt an einer Wirkstoffkombination, bestehend aus Benzodioxolderivaten der Formel I gemäß Anspruch 1 und
   A) Carbamaten, und/oder
   B) Carbonsäureestern und/oder
   C) Phosphorsäureestern und/oder
   D) Cycloalkanen und/oder
   E) Halogenalkanen.

4. Insektizide und akarizide Mittel gemäß Anspurch 3, dadurch gekennzeichnet, daß in der Wirkstoffkombination das Gewichtsverhältnis von Benzodioxolderivaten zu Wirkstoffen zwischen 0,1:10 und 10:0,1 liegt.

5. Verfahren zur Bekämpfung von Insekten und Spinnentieren dadurch gekennzeichnet, daß man eine Wirkstoffkombination gemäß Anspruch 3 oder 4 auf Insekten und Spinnentieren und/oder ihren Lebensraum einwirken läßt.

6. Verwendung von Wirkstoffkombination gemäß Anspruch 3 oder 4 zur Bekämpfung von Insekten und Spinnentieren.

7. Verfahren zur Herstellung von insektiziden und akariziden Mitteln, dadurch gekennzeichnet, daß man eine Wirkstoffkombination gemäß Anspruch 3 oder 4 mit Streckmitteln und/oder oberflächenaktiven Mitteln vermischt.

0000760

Europäisches Patentamt

**EUROPÄISCHER RECHERCHENBERICHT**

Nummer der Anmeldung

EP 78 10 ...

| EINSCHLÄGIGE DOKUMENTE | | | KLASSIFIKATION DER ANMELDUNG (Int.Cl.²) |
|---|---|---|---|
| Kategorie | Kennzeichnung des Dokuments mit Angabe, soweit erforderlich, der maßgeblichen Teile | betrifft Anspruch | |
| | Keine Entgegenhaltungen.<br>---- | | C 07 D 317/54<br>A 01 N 9/24 |
| | | | RECHERCHIERTE SACHGEBIETE (Int. Cl.²) |
| | | | C 07 D 317/54 |
| | | | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument<br>&: Mitglied der gleichen Patentfamilie, übereinstimmendes Dokument |
| X | Der vorliegende Recherchenbericht wurde für alle Patentansprüche erstellt. | | |

| Recherchenort | Abschlußdatum der Recherche | Prüfer |
|---|---|---|
| Den Haag | 10-11-1978 | FRANCOIS |

EPA form 1503.1 06.78